# EUROPEAN PATENT SPECIFICATION

(11) **EP 4 578 390 B1**
(45) Date of publication and mention of the grant of the patent: **16.09.2026**
(21) Application number: 23307441.8
(22) Date of filing: 30.12.2023
(51) Int. Cl.: A61B 5/353, A61B 5/363, A61B 5/00, G16H 50/20

(54) **CARDIAC SIGNAL PROCESSING DEVICE**
VORRICHTUNG ZUR VERARBEITUNG VON HERZSIGNALEN
DISPOSITIF DE TRAITEMENT DE SIGNAL CARDIAQUE

(43) Date of publication of application: 02.07.2025
(73) Proprietor: Substrate HD, 13006 Marseille (FR)
(72) Inventor: DEMARCY, Thomas, 13006 Marseille (FR); VOUNDY, El Makki, 13006 Marseille (FR)
(74) Representative: Plasseraud IP

(56) References cited:
- FR-A1- 3 119 537
- YULDASHEV ZAFAR ET AL: "Processing of Synchronous Recordings of Surface ECG and Intracardiac Potentials for Diagnostics of Dangerous Heart Rate Disturbances", 2019 URAL SYMPOSIUM ON BIOMEDICAL ENGINEERING, RADIOELECTRONICS AND INFORMATION TECHNOLOGY (USBEREIT), IEEE, 25 April 2019 (2019-04-25), pages 102 - 105, XP033561097, DOI: 10.1109/USBEREIT.2019.8736673
- SUNDARARAJAN PRATHIC ET AL: "Automatic Diagnosis of Cardiac Disease from Twelve-Lead and Reduced-Lead ECGs Using Multilabel Classification", 2021 COMPUTING IN CARDIOLOGY (CINC), CREATIVE COMMONS, vol. 48, 13 September 2021 (2021-09-13), pages 1 - 4, XP033999351, DOI: 10.23919/CINC53138.2021.9662899
- KISTLER P M ET AL: "P-Wave Morphology in Focal Atrial Tachycardia", JOURNAL OF THE AMERICAN COLLEGE OF CARDIOLOGY, ELSEVIER, AMSTERDAM, NL, vol. 48, no. 5, 5 September 2006 (2006-09-05), pages 1010 - 1017, XP025147425, ISSN: 0735-1097, [retrieved on 20060905], DOI: 10.1016/J.JACC.2006.03.058

## Description

### Technical Field

The invention relates to the field of cardiac signal processing. More specifically, it finds application in the field of atrial fibrillation treatment.

### Background

In general, the treatment of atrial tachycardia (or "AT", or *"atrial tachycardia"* in English) consists of burning the area of the heart at the origin of the tachycardia. For this purpose, this area should be detected at first.

Most research articles giving an overview on the problem of location of the AT are primarily based on the analysis of the "P waves", a specific portion of the electrocardiogram (hereinafter "ECG") which corresponds to the depolarisation of the auricles.

In 1995, in the article "Use of p wave configuration during atrial tachycardia to predict site of origin", Journal of the American College of Cardiology, 26(5):1315-1324, Tang et al have focused on the analysis of the polarity of the P waves in the surface electrodes to determine which auricle (the right one or the left one) is at the origin of the tachycardia. The derivations aVL and V1 have proven to be the most useful ones to differentiate the right sites from the left sites: a positive P wave in the aVL derivations predicts a right site with a 88% sensitivity and a 79% specificity. The sensitivity and the specificity of a positive P wave in the V1 derivation predicting a left site have been 93% and 88% respectively.

Since then, several studies have extended the analysis of the polarity of the P waves to predict the location of the site with a greater accuracy. In 2006, in the article "P-Wave Morphology in Focal Atrial Tachycardia: Development of an Algorithm to Predict the Anatomic Site of Origin», 48:1010-1017, Kistler et al have studied 130 focal AT to build a decision tree in order to find the origin amongst 11 possible regions, where the nodes are divided according to the polarity of the P waves in the different ECG derivations. This algorithm has succeeded in correctly classifying the origin in 93% of the 30 new ATs.

However, the criteria retained in these articles prevent the use thereof in a real-time context. Indeed, they are based on a posteriori processing allowing detecting the P waves in a very accurate manner, which is not possible in real-time. Based on the P waves determined in real-time, the results would be less conclusive and these methods unsuitable.

The invention improves the situation.

### Summary

To this end, it provides a device for processing cardiac signals, comprising:
- a data storage arranged to receive input data sets each comprising a plurality of P wave segments each associated with an electrocardiogram track and with an acquisition time window, and a plurality of coronary sinus signals associated with the same acquisition time window and having one or more activation sequence(s),
- a random convolutional kernel-based extractor arranged, for a given input data set, to determine an electrocardiogram feature vector, and
- a machine-learning based locator using decision trees trained on data comprising sets of electrocardiogram feature vectors labelled with a value indicating a cardiac region identifier, and arranged to receive an electrocardiogram feature vector from the random convolutional kernel

This device is particularly advantageous because it allows determining in real-time a probable area of the anomaly at the origin of the perpetuation of the AT. This means that a practitioner has the possibility of focussing on a reduced portion of the heart to determine which he/she considers the actual area at the origin of the AT. Thus, the determination of the probable origin area of the AT enables the practitioner to save valuable time, although the latter has neither a confirmed medical sense nor the value of a diagnosis.

According to various embodiments, the invention may have one or more of the following features:
- the random convolutional kernel-based extractor is a ROCKET classifier,
- the random convolutional kernel-based extractor is a Mini-ROCKET classifier,
- the machine-learning based locator is arranged to implement a random-forest classifier,
- the machine-learning based locator is arranged to implement an XGB or LightGBM machine learning model, and
- the locator is arranged to receive as input an electrocardiogram feature vector comprising more than 1000 values.

According to a second aspect, a method for determining spatiotemporal dispersion in electrograms is provided. This method for processing cardiac signals comprises the following operations:
a) receiving input data sets each comprising a plurality of P wave segments each associated with an electrocardiogram track and with an acquisition time window, and a plurality of coronary sinus signals associated with the same acquisition time window and having one or more activation sequence(s),
b) determining an electrocardiogram feature vector by feeding the input data sets of operation a) as inputs to a random convolutional kernel-based extractor,
c) providing said electrocardiogram feature vector of operation b) as input of a machine-learning based locator using decision trees, and returning a cardiac region identifier as output.

In various embodiments, this method may present one or more of the following features:
- operation b) includes using a ROCKET classifier as said random convolutional kernel-based extractor,
- operation b) includes using a Mini-ROCKET classifier as said random convolutional kernel-based extractor,
- operation c) includes using a random-forests classifier as said machine-learning based locator, and
- operation c) includes using an XGB or LightGBM machine learning classifier as said machine-learning based locator.

According to a third aspect, a computer program is provided. The computer program includes instructions for adapting an apparatus to perform the method of any one of the embodiments of the second aspect.

According to a fourth aspect, a carrier containing a computer program that includes instructions for adapting an apparatus to perform the method of any one of the embodiments of the second aspect is provided. In some embodiments, the carrier is one of an electronic signal, optical signal, radio signal, or computer readable storage medium.

According to a fifth aspect, an apparatus is provided. The apparatus includes processing circuitry and a memory containing instructions executable by the processing circuitry that causes the apparatus to perform the method of any one of the embodiments of the second aspect.

Other features and advantages of the invention will appear better upon reading the following description, with reference to examples given for illustrative and non-limiting purposes, with reference to the drawings wherein:
- Figure 1 shows a schematic diagram of a device according to the invention,
- Figure 2 shows an example of modules used in the computer program code of Figure 1,
- Figure 3 shows an example of a function executed by the device of Figure 1, and
- Figure 4 shows the division of a human heart into 21 regions.

The drawings and the description hereinafter essentially contain elements of certain nature. Hence, they could not only serve to better understand the present invention, but also contribute to the definition thereof, where appropriate.

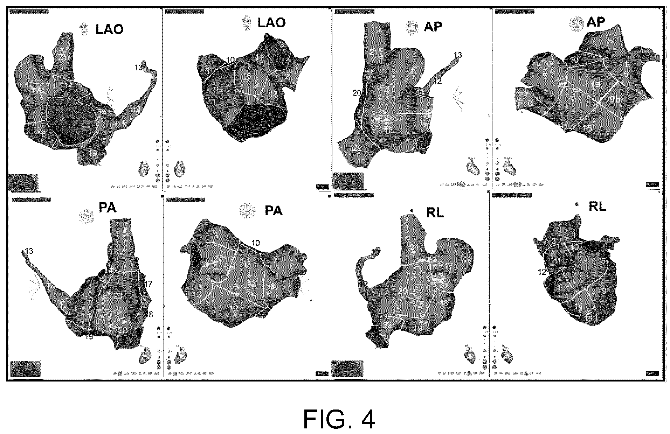

In an attempt to classify the origin of the AT, the Applicant has for a long time worked based on a division of the auricles into 21 areas as shown on Figure 4. Throughout these works, and searching for a solution that could be used in real-time, the Applicant has found that it was relevant to group these areas into 4 groups: the left auricle (areas 7, 8, 9A, 9B, 10, 11A, 11B and 12), the right auricle (areas 18, 19, 20B, 20H, and 22), the septum (areas 5, 6, 14, 15, 17 and 21), and the lateral portion of the left auricle (areas 1, 2, 3, 4, 13 and 16).

As this will be seen hereinbelow, the device of the invention allows determining, for a set of ECG signals and an intracardiac reference catheter commonly placed in the vein of the coronary sinus, which will hereafter be referred to by the expression "CS", the group that contains the origin of the AT. This determination is particularly interesting because it offers a starting point to the practitioner to search and determine the specific origin area of the AT and therefore the diseased portion of the heart. Indeed, if these groups define 4 larger areas than the 21 original areas, this is already enough to confer a great advantage on the procedure.

Furthermore, the combination of ECG signals and of signals derived from the coronary sinus is particularly innovative. It has never been disclosed before, and it enables the use of machine learning, which enables the operation of the device 2 in real-time.

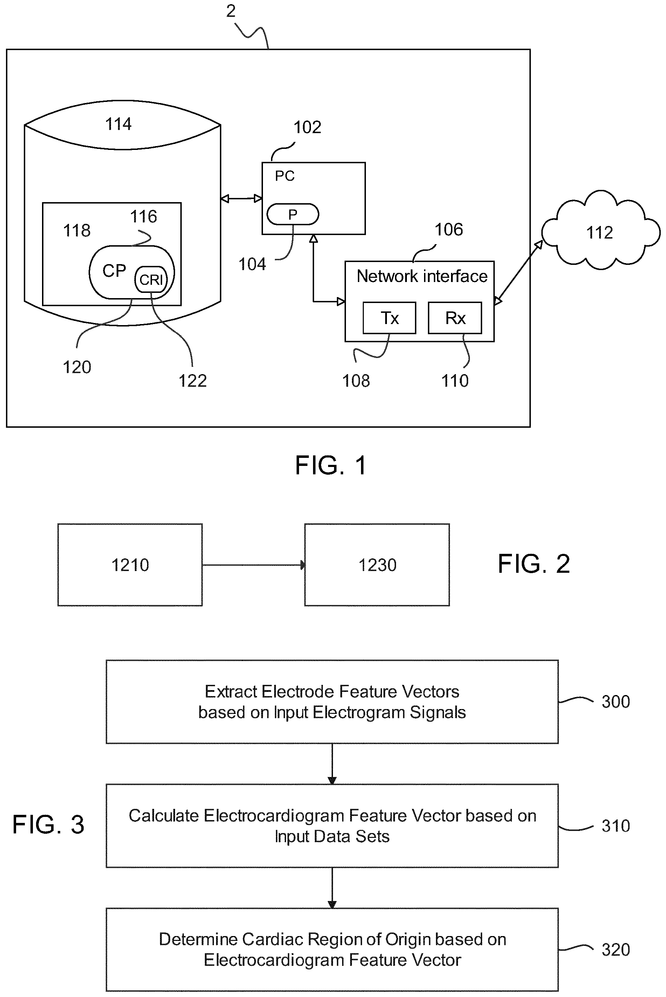

Figure 1 shows a general diagram on an embodiment of the computer device according to the invention.

The computer device shown on Figure 1 as a block diagram of an apparatus 2 (*e.g.,* a network node, connected device, and the like), according to an embodiment. As shown in Figure 1 the apparatus may comprise: processing circuitry (PC) 102, which may include one or more processors (P) or processing circuitry 104; a network interface 106 comprising a transmitter (Tx) 108 and a receiver (Rx) 110 for enabling the apparatus to transmit data to and receive data from other computing devices connected to a network 112 (e.g., an Internet Protocol (IP) network) to which network interface 106 is connected; and a local storage unit (a.k.a., "data storage system") 114, which may include one or more non-volatile storage devices and/or one or more volatile storage devices.

In embodiments where PC 102 includes a programmable processor, a computer program product (CPP) 116 may be provided. CPP 116 includes a computer readable medium (CRM) 118 storing a computer program (CP) 120 comprising computer readable instructions (CRI) 122. CRM 118 may be a non-transitory computer readable medium, such as, magnetic media (e.g., a hard disk), optical media, memory devices (e.g., random access memory, flash memory), and the like.

The one or more processors or processing circuitry 104 include any means known for performing automated calculus, such as CPUs, GPUs, CPUs and/or GPUs grids, remote calculus grids, specifically configured FPGAs, specifically configured ASICs, specialized chips such as SOCs or NOCs, AI specialized chips, etc.

In an embodiment, data storage 114 stores input data sets as input. Each input data set comprises a plurality of P wave segments each associated with an electrocardiogram track and with an acquisition time window, and a plurality of segments deduced from coronary sinus signals associated with the same acquisition time window. In concrete terms, this means that a time window, typically 10 seconds before a current measurement time, is used to split an ECG with 9 tracks (deduced from a 12-track sensor 3 of which are ignored as they correspond to a linear combination of some of the other tracks) as wells as 5 CS signal tracks. In the particular case of the ECG, only the segments corresponding to a P wave are used. Since each heart rate is particular, this means that the input data set receives a variable number of P wave segments each being deduced from an ECG track. The present invention does not cover the particular method for obtaining the P wave segments and assumes that these form an input. In some embodiments, the apparatus 2 could be arranged to directly analyse the ECG tracks and to determine the P wave segments therein. By P wave segment, it should be understood that subsets of each ECG track are split inside the time window and define a segment of the latter.

As shown hereinbelow, all of the P wave segments of each data set are processed by an extractor module in order to deduce features therefrom which serve as an input to a locator module. Thus, for each input data set, an electrocardiogram feature vector comprising several thousand features will be generated.

Comparatively, the Applicant previous invention, which is the object of a patent family based on French patent application FR2208851, discloses a feature extractor which provides 58 features. The features were chosen and tuned for their medical meaning and relevance with respect to atrial tachycardia. By contrast, the electrocardiogram feature vector may comprise over 10000 features, which do not appear to have a medical meaning *per se.* However, this high number of automatically generated features captures even more precisely the phenomena in the electrocardiogram and the CS.

In the example described herein, the data storage 114 may be realized in any way suitable, that is by means of a hard disk drive, a solid-state drive, a flash memory, a memory embedded in a processor, a distant storage accessible in the cloud, etc. Data storage 114 may also store any transitory data which may be generated in the course of executing the invention, as well as data resulting from the operation of the apparatus 2, possibly combined with practitioner made annotations.

Figure 2 shows an exemplary schematic of computer program 120.

The computer program (CP) 120 comprises two main modules which work hand-in-hand:
- An extractor module which may be implemented with a random convolutional kernel based feature extractor 1210, and
- A locator module which may be implemented with a gradient-boosted based machine learning module 1230.

In the example described herein, the random convolutional kernel based feature extractor 1210 is based on the framework ROCKET described in the article by Dempster et al. "ROCKET: exceptionally fast and accurate time series classification using random convolutional kernels", Data Min Knowl Disc 34, 1454-1495 (2020), https://doi.org/10.1007/s10618-020-00701-z.

ROCKET is a machine learning algorithm for feature extraction. It generates random convolutional kernels (random length, weights, dilation, and padding) and random thresholds (called bias) which can transform multivariate time series into high-dimensional feature vectors. It can capture complex patterns and relationships.

The Applicant has also found that a multivariate alternative of ROCKET, named MiniROCKET can be even more efficient, as the multivariate random feature generation can capture relationships between specific tracks, *e.g.* extract temporal relationships such as delay between the tracks of the CS. MiniROCKET is described in the article by Dempster et al. "MINIROCKET: A Very Fast (Almost) Deterministic Transform for Time Series Classification", KDD '21: Proceedings of the 27th ACM SIGKDD Conference on Knowledge Discovery & Data MiningAugust 2021, Pages 248-257, https://doi.org/10.1145/3447548.3467231.

The random convolutional kernel based feature extractor 1210 receives the input data set comprising a plurality of P wave segments each associated with an electrocardiogram track and with an acquisition time window, and a plurality of segments deduced from coronary sinus signals associated with the same acquisition time window in the data storage 114, and it returns an electrocardiogram feature vector.

As mentioned above, the electrocardiogram feature vector may contain more than 1000 and as many as 10000 features. While conventional methods involve manual feature design and parameter tuning, embodiments disclosed herein enable generating a large number of features and uses a data-driven approach to extract the most relevant features that can adapt to the different characteristics of the signal.

The large number of generated features is possible because the convolutional operations performed by the random kernels can be efficiently implemented in parallel, taking advantage of modern hardware architectures, such as GPUs or parallel processing units. Due to the specificities of the electrocardiogram and CS signals, the ROCKET framework is very well suited to extract all information, and the patterns identified by the extractor module can include distinctive shapes and configurations associated with different polarities of signals. The convolution transformations applied can easily magnify features related to the amplitude (extrema, prominence, etc.) and the shape (bipolarity, isoline, etc.) of the input signals. As mentioned above, the multivariate random feature generation can capture relationships between specific tracks, *e.g*. extract temporal relationships such as delay between the tracks of the CS. In the same vein, the extrema values, transformation of their values, polarity profile, and various temporal relationships (such as those determined using DTW) used in FR2208851 as explicit features are captured by the features generated automatically.

The electrocardiogram feature vector generated by the extractor module 1210 is provided as an input vector to the locator module 1230. As mentioned above, the locator module 1230 may be implemented by a random-forest classifier that has been trained with a set of training data comprised of electrocardiogram feature vectors in which the output origin area was known, i.e. the training data set contained electrocardiogram feature vectors labelled with cardiac region identifiers. Thus, once the training of the machine learning model is performed, the origin area can be determined in real-time in inference mode. Alternatively, the locator module 1230 could implement a machine learning model using decision trees different from random forests, for example based on gradient boosting , or extreme gradient boosting (known as "XGBoost", see the article by Chen et al. "XGBoost: A Scalable Tree Boosting System", In Proceedings of the 22nd ACM SIGKDD International Conference on Knowledge Discovery and Data Mining (KDD '16), Association for Computing Machinery, New York, NY, USA, 785-794. https://doi.org/10.1145/2939672.2939785), or LightGBM (see the article by Ke et al. "LightGBM: A highly efficient gradient boosting decision tree", Advances in Neural Information Processing Systems, 30, 3146-3154).

Figure 3 shows an example of an implementation of a function executed by the apparatus 2 to determine the cardiac region identifier from which an atrial tachycardia originates based on a patient's electrocardiogram and CS signals.

In a first operation 300, the electrocardiogram and CS signals are analyzed in order to detect the plurality of P wave segments each associated with an electrocardiogram track and with an acquisition time window, and the plurality of coronary sinus signals associated with the same acquisition time window and having one or more activation sequence(s). In some embodiments, operation 300 is performed out of the apparatus 2, and the apparatus 2 directly receives input data sets as inputs.

The input data sets are thereafter provided to the random convolutional kernel-based extractor 1210 as inputs. In an operation 310, the random convolutional kernel-based extractor 1210 determines and returns an electrocardiogram feature vector.

Finally, in an operation 320, the electrocardiogram feature vector of operation 310 is fed as input to the machine-learning based locator 1230, which returns an AT cardiac region of origin identifier.

The tests of the Applicant have shown that the device 2 allows obtaining even more satisfactory results than the implementation of French patent application FR2208851.

In comparing methods based on random convolution kernels to those based on manually designed features on a database containing 14 hours of annotated signals related to the mechanism of AT after persistent atrial fibrillation ablation, where the task was to predict and classify "left," "right," or "septum,", a 5% improvement in precision and a 19% increase in recall were observed.

This database poses a particular challenge due to over a third of patients exhibiting either low-amplitude P-waves or having them obscured by T-waves.

Thus, the device 2 allows carrying out a "high-level" determination of the origin area of an AT in real-time, which allows considerably accelerating ablation procedures in the context of AT treatment and reduces the risks of error related to the exploration of "useless" areas of the heart, i.e. areas that are not likely to be at the origin of the AT.

While various embodiments of the present disclosure are described herein, it should be understood that they have been presented by way of example only, and not limitation. Thus, the breadth and scope of the present disclosure should not be limited by any of the above-described exemplary embodiments.

In this computing device, the identified cardiac region of interest may include the region of a heart that contains the atrial tachycardia.

Additionally, while the processes described above and illustrated in the drawings are shown as a sequence of steps, this was done solely for the sake of illustration. Accordingly, it is contemplated that some steps may be added, some steps may be omitted, the order of the steps may be re-arranged, and some steps may be performed in parallel.

## Claims

1. A device for processing cardiac signals, comprising:
- a data storage (114) arranged to receive input data sets each comprising a plurality of P wave segments each associated with an electrocardiogram track and with an acquisition time window, and a plurality of coronary sinus signals associated with the same acquisition time window and having one or more activation sequence(s),
- a random convolutional kernel-based extractor (1210) arranged, for a given input data set, to determine an electrocardiogram feature vector, and
- a machine-learning based locator (1230) using decision trees trained on data comprising sets of electrocardiogram feature vectors labelled with a value indicating an atrial tachycardia cardiac region of origin identifier, and arranged to receive an electrocardiogram feature vector from the random convolutional kernel-based extractor (1230) as input, and to return a cardiac region identifier as output.

2. The device according to claim 1, in which the random convolutional kernel-based extractor (1210) is a ROCKET classifier.

3. The device according to claim 1, in which the random convolutional kernel-based extractor (1210) is a Mini-ROCKET classifier.

4. The device according to one of the preceding claims, in which the machine-learning based locator (1230) is arranged to implement a random-forest classifier.

5. The device according to one of claims 1 to 3, in which the machine-learning based locator (1230) is arranged to implement an XGB or LightGBM machine learning model.

6. The device according to one of the preceding claims, in which the machine-learning based locator (1230) is arranged to receive as input an electrocardiogram feature vector comprising more than 1000 values.

7. A method for processing cardiac signals comprising the following operations:
a) receiving input data sets each comprising a plurality of P wave segments each associated with an electrocardiogram track and with an acquisition time window, and a plurality of coronary sinus signals associated with the same acquisition time window and having one or more activation sequence(s),
b) determining (310) an electrocardiogram feature vector by feeding the input data sets of operation a) as inputs to a random convolutional kernel-based extractor,
c) providing (320) said electrocardiogram feature vector of operation b) as input of a machine-learning based locator using decision trees, and returning a cardiac region identifier as output.

8. The method according to claim 7 , in which operation b) includes using a ROCKET classifier as said random convolutional kernel-based extractor.

9. The method according to claim 7, in which operation b) includes using a Mini-ROCKET classifier as said random convolutional kernel-based extractor.

10. The method according to one of claims 7 to 9, in which operation c) includes using a random-forests classifier as said machine-learning based locator.

11. The method according to one of claims 7 to 9, in which operation c) includes using an XGB or LightGBM machine learning classifier as said machine-learning based locator.

12. A computer program (120) comprising instructions (122) which, when executed by processing circuitry (102), causes the processing circuitry to carry out the methods of any one of claims 7 to 11.

13. A carrier containing a computer program (120) according to claim 12, in which the carrier comprises one of an electronic signals, optical signal, radio signal or computer readable storage medium.

14. A computer program product (116) comprising a non-transitory computer readable medium (118) having stored thereon a computer program (120) according to claim 13.

## Patentansprüche

1. Vorrichtung zur Verarbeitung von Herzsignalen, umfassend:
- einen Datenspeicher (114), der so eingerichtet ist, dass er Eingangsdatensätze empfängt, die jeweils eine Vielzahl von P-Wellen-Segmenten, die jeweils einer Elektrokardiogramm-Spur und einem Erfassungszeitfenster zugeordnet sind, und eine Vielzahl von Koronarsinus-Signalen umfassen, die demselben Erfassungszeitfenster zugeordnet sind und eine oder mehrere Aktivierungssequenz(en) aufweisen,
- einen Extraktor auf Basis zufälliger Faltungskerne (1210), der so eingerichtet ist, dass er für einen gegebenen Eingangsdatensatz einen Elektrokardiogramm-Merkmalsvektor bestimmt, und
- einen Lokalisierer auf Basis maschinellen Lernens (1230), der Entscheidungsbäume verwendet, die auf Daten trainiert sind, die Sätze von Elektrokardiogramm-Merkmalsvektoren umfassen, die mit einem Wert markiert sind, der eine Kennung für den kardialen Ursprungsbereich einer atrialen Tachykardie angibt, und der so eingerichtet ist, dass er einen Elektrokardiogramm-Merkmalsvektor von dem Extraktor auf Basis zufälliger Faltungskerne (1230) als Eingang empfängt und eine Kennung für einen kardialen Bereich als Ausgang zurückgibt.

2. Vorrichtung nach Anspruch 1, wobei der Extraktor auf Basis zufälliger Faltungskerne (1210) ein ROCKET-Klassifikator ist.

3. Vorrichtung nach Anspruch 1, wobei der Extraktor auf Basis zufälliger Faltungskerne (1210) ein Mini-ROCKET-Klassifikator ist.

4. Vorrichtung nach einem der vorhergehenden Ansprüche, wobei der Lokalisierer auf Basis maschinellen Lernens (1230) so eingerichtet ist, dass er einen Random-Forest-Klassifikator implementiert.

5. Vorrichtung nach einem der Ansprüche 1 bis 3, wobei der Lokalisierer auf Basis maschinellen Lernens (1230) so eingerichtet ist, dass er ein XGB- oder LightGBM-Maschinenlernmodell implementiert.

6. Vorrichtung nach einem der vorhergehenden Ansprüche, wobei der Lokalisierer auf Basis maschinellen Lernens (1230) so eingerichtet ist, dass er als Eingang einen Elektrokardiogramm-Merkmalsvektor empfängt, der mehr als 1000 Werte umfasst.

7. Verfahren zur Verarbeitung von Herzsignalen, das die folgenden Operationen umfasst:
a) Empfangen von Eingangsdatensätzen, die jeweils eine Vielzahl von P-Wellen-Segmenten, die jeweils einer Elektrokardiogramm-Spur und einem Erfassungszeitfenster zugeordnet sind, und eine Vielzahl von Koronarsinus-Signalen umfassen, die demselben Erfassungszeitfenster zugeordnet sind und eine oder mehrere Aktivierungssequenz(en) aufweisen,
b) Bestimmen (310) eines Elektrokardiogramm-Merkmalsvektors durch Zuführen der Eingangsdatensätze aus Operation a) als Eingänge in einen Extraktor auf Basis zufälliger Faltungskerne,
c) Bereitstellen (320) des besagten Elektrokardiogramm-Merkmalsvektors aus Operation b) als Eingang eines Lokalisierers auf Basis maschinellen Lernens unter Verwendung von Entscheidungsbäumen und Zurückgeben einer Kennung für einen kardialen Bereich als Ausgang.

8. Verfahren nach Anspruch 7, wobei Operation b) die Verwendung eines ROCKET-Klassifikators als besagten Extraktor auf Basis zufälliger Faltungskerne einschließt.

9. Verfahren nach Anspruch 7, wobei Operation b) die Verwendung eines Mini-ROCKET-Klassifikators als besagten Extraktor auf Basis zufälliger Faltungskerne einschließt.

10. Verfahren nach einem der Ansprüche 7 bis 9, wobei Operation c) die Verwendung eines Random-Forest-Klassifikators als besagten Lokalisierer auf Basis maschinellen Lernens einschließt.

11. Verfahren nach einem der Ansprüche 7 bis 9, wobei Operation c) die Verwendung eines XGB- oder LightGBM-Maschinenlern-Klassifikators als besagten Lokalisierer auf Basis maschinellen Lernens einschließt.

12. Computerprogramm (120), umfassend Anweisungen (122), die bei Ausführung durch eine Verarbeitungsschaltung (102) die Verarbeitungsschaltung veranlassen, die Verfahren nach einem der Ansprüche 7 bis 11 auszuführen.

13. Träger, der ein Computerprogramm (120) nach Anspruch 12 enthält, wobei der Träger eines von einem elektronischen Signal, einem optischen Signal, einem Funksignal oder einem computerlesbaren Speichermedium umfasst.

14. Computerprogrammprodukt (116), das ein nicht-flüchtiges computerlesbares Medium (118) umfasst, auf dem ein Computerprogramm (120) nach Anspruch 13 gespeichert ist.

## Revendications

1. Dispositif de traitement de signaux cardiaques, comprenant :
- un stockage de données (114) agencé pour recevoir des ensembles de données d'entrée comprenant chacun une pluralité de segments d'onde P associés chacun à une piste d'électrocardiogramme et à une fenêtre temporelle d'acquisition, et une pluralité de signaux de sinus coronaire associés à la même fenêtre temporelle d'acquisition et ayant une ou plusieurs séquence(s) d'activation,
- un extracteur à base de noyaux de convolution aléatoires (1210) agencé, pour un ensemble de données d'entrée donné, pour déterminer un vecteur de caractéristiques d'électrocardiogramme, et
- un localisateur à base d'apprentissage automatique (1230) utilisant des arbres de décision entraînés sur des données comprenant des ensembles de vecteurs de caractéristiques d'électrocardiogramme étiquetés avec une valeur indiquant un identifiant de région cardiaque d'origine de tachycardie atriale, et agencé pour recevoir en entrée un vecteur de caractéristiques d'électrocardiogramme provenant de l'extracteur à base de noyaux de convolution aléatoires (1230), et pour renvoyer en sortie un identifiant de région cardiaque.

2. Dispositif selon la revendication 1, dans lequel l'extracteur à base de noyaux de convolution aléatoires (1210) est un classifieur ROCKET.

3. Dispositif selon la revendication 1, dans lequel l'extracteur à base de noyaux de convolution aléatoires (1210) est un classifieur Mini-ROCKET.

4. Dispositif selon l'une des revendications précédentes, dans lequel le localisateur à base d'apprentissage automatique (1230) est agencé pour mettre en œuvre un classifieur à forêts aléatoires.

5. Dispositif selon l'une des revendications 1 à 3, dans lequel le localisateur à base d'apprentissage automatique (1230) est agencé pour mettre en œuvre un modèle d'apprentissage automatique XGB ou LightGBM.

6. Dispositif selon l'une des revendications précédentes, dans lequel le localisateur à base d'apprentissage automatique (1230) est agencé pour recevoir en entrée un vecteur de caractéristiques d'électrocardiogramme comprenant plus de 1000 valeurs.

7. Procédé de traitement de signaux cardiaques comprenant les opérations suivantes :
a) la réception d'ensembles de données d'entrée comprenant chacun une pluralité de segments d'onde P associés chacun à une piste d'électrocardiogramme et à une fenêtre temporelle d'acquisition, et une pluralité de signaux de sinus coronaire associés à la même fenêtre temporelle d'acquisition et ayant une ou plusieurs séquence(s) d'activation,
b) la détermination (310) d'un vecteur de caractéristiques d'électrocardiogramme en fournissant les ensembles de données d'entrée de l'opération a) comme entrées à un extracteur à base de noyaux de convolution aléatoires,
c) la fourniture (320) dudit vecteur de caractéristiques d'électrocardiogramme de l'opération b) comme entrée d'un localisateur à base d'apprentissage automatique utilisant des arbres de décision, et le renvoi en sortie d'un identifiant de région cardiaque.

8. Procédé selon la revendication 7, dans lequel l'opération b) inclut l'utilisation d'un classifieur ROCKET en tant que ledit extracteur à base de noyaux de convolution aléatoires.

9. Procédé selon la revendication 7, dans lequel l'opération b) inclut l'utilisation d'un classifieur Mini-ROCKET en tant que ledit extracteur à base de noyaux de convolution aléatoires.

10. Procédé selon l'une des revendications 7 à 9, dans lequel l'opération c) inclut l'utilisation d'un classifieur à forêts aléatoires en tant que ledit localisateur à base d'apprentissage automatique.

11. Procédé selon l'une des revendications 7 à 9, dans lequel l'opération c) inclut l'utilisation d'un classifieur à apprentissage automatique XGB ou LightGBM en tant que ledit localisateur à base d'apprentissage automatique.

12. Programme d'ordinateur (120) comprenant des instructions (122) qui, lorsqu'elles sont exécutées par une circuiterie de traitement (102), amènent la circuiterie de traitement à mettre en œuvre les procédés de l'une quelconque des revendications 7 à 11.

13. Support contenant un programme d'ordinateur (120) selon la revendication 12, dans lequel le support comprend l'un parmi un signal électronique, un signal optique, un signal radio ou un support de stockage lisible par ordinateur.

14. Produit-programme d'ordinateur (116) comprenant un support non transitoire lisible par ordinateur (118) sur lequel est stocké un programme d'ordinateur (120) selon la revendication 13.
